# EUROPEAN PATENT SPECIFICATION

(11) **EP 3 560 437 B1**
(45) Date of publication and mention of the grant of the patent: **23.02.2022**
(21) Application number: 19170302.4
(22) Date of filing: 18.04.2019
(51) Int. Cl.: A61B 17/115

(54) **STAPLING DEVICE WITH CUT RING BIASING MEMBER**
KLAMMERVORRICHTUNG MIT SCHNEIDRINGVORSPANNELEMENT
DISPOSITIF D'AGRAFAGE AYANT UN ÉLÉMENT DE SOLLICITATION DE BAGUE DE COUPE

(30) Priority: 23.04.2018 US 201862661144 P; 13.03.2019 US 201916351894
(43) Date of publication of application: 30.10.2019
(73) Proprietor: Covidien LP, Mansfield, MA 02048 (US)
(72) Inventor: Williams, Justin, Southbury, CT 06488 (US)
(74) Representative: Maschio, Antonio

(56) References cited:
- EP-A1- 2 545 859
- EP-A2- 0 594 436
- EP-A2- 2 614 785
- EP-A2- 2 774 551
- US-A1- 2005 021 053
- US-A1- 2005 205 639
- US-A1- 2010 163 598

## Description

### CROSS-REFERENCE TO RELATED APPLICATIONS

This application claims the benefit of and priority to U.S. Provisional Patent Application No. 62/661,224 filed April 23, 2018.

### BACKGROUND

### 1. Technical Description

The present disclosure is directed to circular stapling devices and more particularly, to a shell assembly of a circular device with structure to facilitate separation of a knife and a cut ring after firing.

### 2. Background of Related Art

Circular stapling devices for performing end-to-end or end-to-side anastomosis procedures are well known. Typically, circular stapling devices include a staple cartridge that supports a plurality of staples, a pusher that is movable in relation to the staple cartridge to eject the staples from the staple cartridge, an annular knife that is movable to core tissue within the anastomosis, and an anvil assembly that is positioned to deform the staples. In embodiments, the anvil includes a cut ring that provides a back stop for the annular knife.

In known stapling devices, the anvil assembly includes an anvil head that is pivotal from an operative position to a tilted position to reduce a profile of the anvil assembly for subsequent removal of the anvil assembly from a patient. In such stapling devices, the cut ring is movable in response to engagement by the annular knife from a first position that prevents movement of the anvil head from the operative position to the tilted position to a second position that allows for pivotal movement of the anvil head from the operative position to the tilted position. When the annular knife is advanced into the cut ring of the anvil assembly during firing of the stapling device, the annular knife urges the cut ring from the first position to the second position to facilitate tilting of the anvil head.

Typically, the cut ring is formed in part from a material that is softer than the annular knife such that the annular knife penetrates the cut ring during firing of the stapling device. In some instances, the annular knife sticks to the cut ring and the cut ring is moved back to its first position when the annular knife is retracted to a pre-fired position after firing. When this happens, the anvil head is prevented from moving to the tilted position after firing.

A continuing need exists in the art for an improved shell assembly for a circular stapling device that includes a simple device or mechanism that ensures separation of the annular knife and cut ring after firing of the stapling device to facilitate movement of the anvil head to the tilted position during removal of the stapling device from a patient.

Document US2005205639 A1 describes a circular stapler with an anvil assembly including a cut ring assembly and a backup plate. A retaining clip is positioned in a transverse slot formed in a post of the anvil and comprises a pair of outwardly biased flexible arms. The flexible arms prevent the cut ring assembly and the backup plate from sticking to the knife when the anvil assembly is moved away from the staple housing portion of the surgical stapling device.

### SUMMARY

One embodiment of the present invention, as defined by claim 1, is directed to a circular stapling device having an elongate body, a shell assembly, and an anvil assembly. The elongate body has a proximal portion and a distal portion. The shell assembly includes a housing, a staple cartridge supported on the housing, an annular knife supported within the housing, and a biasing member supported within the housing. The biasing member has a proximal portion and a distal portion. The annular knife is movable within the housing from a retracted position to an advanced position and extends from the distal portion of the housing in the advanced position. The biasing member extends to a position distally of the annular knife when the annular knife is in the retracted position. The anvil assembly includes a center rod and an anvil head that supports a cut ring that is axially aligned with the annular knife. The anvil assembly is movably supported in relation to the shell assembly between an unapproximated position and an approximated position, wherein in the approximated position, the biasing member is engaged with the cut ring and urges the cut ring in a distal direction.

Another embodiment of the present invention, as defined in claim 9, is directed to a shell assembly including a housing, a staple cartridge, an annular knife, and a biasing member. The housing has a proximal portion and a distal portion and defines an annular channel. The staple cartridge is supported on the housing. The shell assembly defines a plurality of staple receiving pockets that support staples. The annular knife is supported within the housing and is movable within the housing from a retracted position to an advanced position. The annular knife extends from the distal portion of the housing when the annular knife is in the advanced position. The biasing member is supported within the annular channel of the housing and extends to a position distally of the annular knife when the annular knife is in the retracted position.

The biasing member is a coil spring.

In some embodiments, the shell assembly includes a pusher assembly including a pusher having a plurality of fingers, wherein the pusher is movable from a retracted position to an advanced position to eject staples from the staple cartridge.

In certain embodiments, the shall assembly includes a knife carrier that supports the annular knife and is movable from a retracted position to an advanced position within the housing, wherein the annular knife engages the cut ring when the anvil assembly and the shell assembly are in the approximated position and the annular knife is in the advanced position.

In embodiments, the annular knife includes a cutting edge that is positioned distally of the biasing member when the annular knife is in the advanced position.

In some embodiments, the biasing member is secured to the knife carrier.

In certain embodiments, the knife carrier defines a retaining groove that receives a coil of the coil spring to secure the biasing member to the knife carrier.

In embodiments, the stapling device includes a handle assembly and the elongate body extends distally from the handle assembly.

In some embodiments, the shell assembly includes a knife carrier that supports the annular knife, and the knife carrier is movable from a retracted position to an advanced position within the housing independently of the pusher.

In certain embodiments, the housing defines an annular channel, and the pusher and the knife carrier are movably supported within the annular channel.

In embodiments, the biasing member is positioned within the annular knife.

### BRIEF DESCRIPTION OF THE DRAWINGS

Various embodiments of the presently disclosed circular stapling device are described herein below with reference to the drawings, wherein:
FIG. 1 is a side perspective view of an exemplary embodiment of the presently disclosed circular stapling device in an unapproximated position;
FIG. 2 is an enlarged view of the indicated area of detail shown in FIG. 1;
FIG. 2A is a cross-sectional view taken along section line 2A-2A of FIG. 2;
FIG. 3 is a side perspective view from the distal end of the shell assembly of the circular stapling device shown in FIG. 1;
FIG. 4 is a side perspective view of the knife carrier assembly of the circular stapling device shown in FIG. 1 with the annular knife removed;
FIG. 5 is a side cross-sectional view of the knife carrier assembly of the circular stapling device shown in FIG.1;
FIG. 6 is an exploded view of the knife carrier assembly shown in FIG. 5; and
FIG. 7 is a side cross-sectional view of the shell assembly of the circular stapling device shown in FIG. 1 with the tool assembly in an approximated, fired condition.

### DETAILED DESCRIPTION OF EMBODIMENTS

The presently disclosed circular stapling device will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "endoscopic" is used generally used to refer to endoscopic, laparoscopic, arthroscopic, and/or any other procedure conducted through small diameter incision or cannula. Further, the term "clinician" is used generally to refer to medical personnel including doctors, nurses, and support personnel.

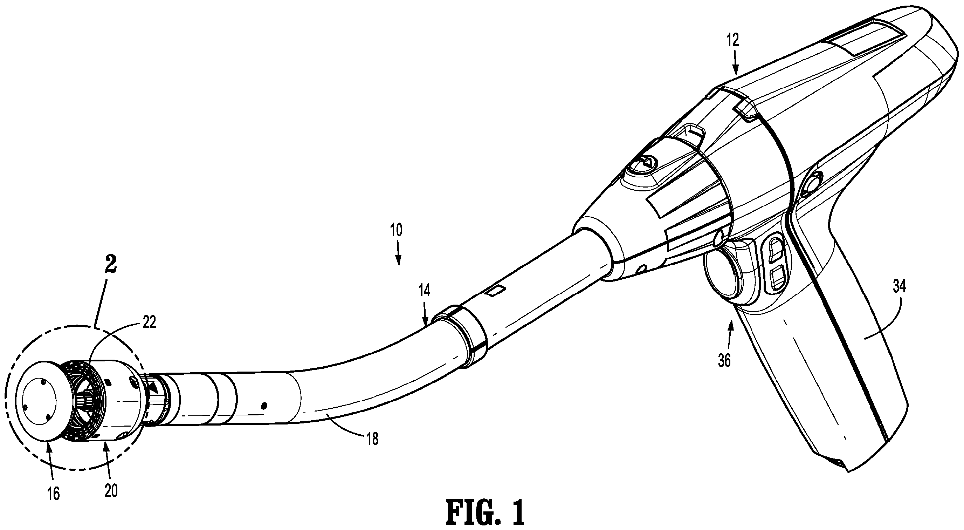

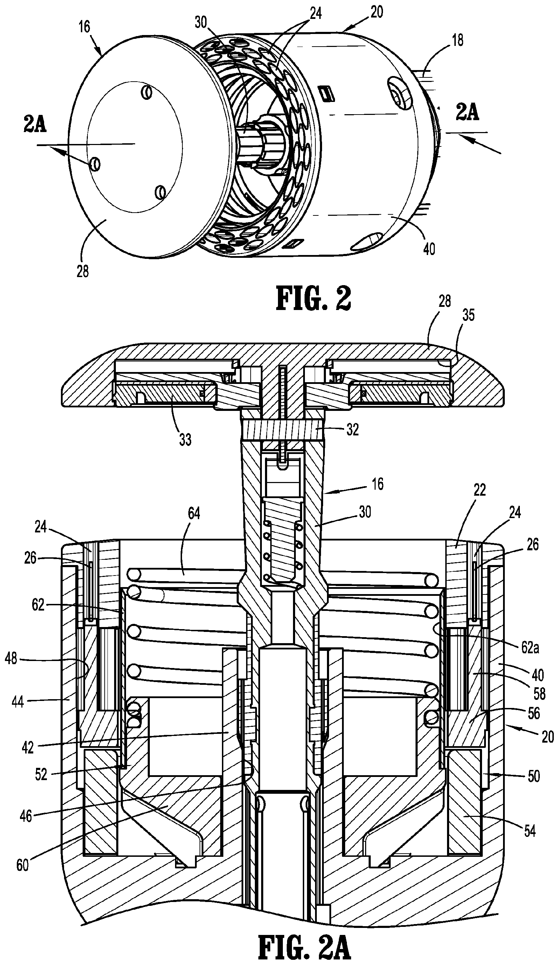

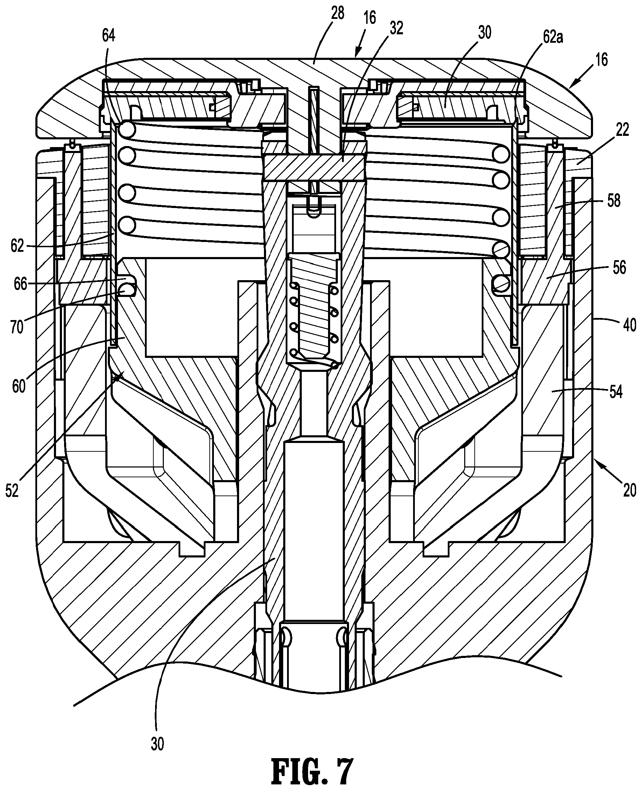

FIGS.1-2A illustrate an exemplary embodiment of the presently disclosed circular stapling device shown generally as stapling device 10. The stapling device 10 includes a handle assembly 12, an elongate body or adaptor assembly 14, and an anvil assembly 16. The adaptor assembly 14 includes an elongate body 18 and a shell assembly 20. The shell assembly 20 has a proximal portion supported on a distal portion of the elongate body 18 and a distal portion that supports a staple cartridge 22. The staple cartridge 22 defines a plurality of annular rows of staple receiving pockets 24. Each of the staple receiving pockets 24 receives a staple 26 (FIG. 2A) such that the staple cartridge 20 supports annular rows of staples 26. The anvil assembly 16 includes a head 28 and a center rod 30. The anvil head 28 is supported on a distal end of the anvil center rod 30 by a pivot member 32 (FIG. 2A) and supports a cut ring 33 (FIG. 2A). The cut ring 33 is movable within a recess 35 (FIG. 2A) defined within the anvil head 28 from a retracted position (FIG. 2A) to an advanced position (FIG. 7.) As is known in the art, in the retracted position, the cut ring 33 prevents tilting of the anvil head 28 in relation to the center rod 30, and in the advanced position, the cut ring 33 allows tilting of the anvil head 28 in relation to the center rod 30. See, e.g., U.S. Patent No. 7,303,106 ("the '106 Patent") for a detailed description of exemplary embodiments of an anvil assembly with a tiltable anvil assembly.

The handle assembly 12 is illustrated as a powered assembly and includes a stationary grip 34 (FIG. 1) and actuation buttons 32 for controlling operation of stapling device functions including approximation of the anvil and shell assemblies 16, 20, respectively, and firing of staples 26 (FIG. 2A) from the staple cartridge 22 of the shell assembly 20. The adaptor assembly 14 is coupled to the handle assembly 12 to translate power from the handle assembly 12 to the anvil and shell assemblies 16, 20. Although the present disclosure illustrates powered handle and adaptor assemblies 12, 14, respectively, it is envisioned that the advantages of the present disclosure as described in detail below are also applicable to circular stapling devices having manually operated handle and adaptor assemblies. The '106 Patent discloses an example of a surgical stapling device including a manually actuated handle assembly and is incorporated herein by reference in its entirety. U.S. Patent Nos. 9,023,014 ("the '014 Patent") and 9,055,943 ("the '943 Patent") disclose examples of surgical stapling devices including exemplary powered handle and adaptor assemblies.

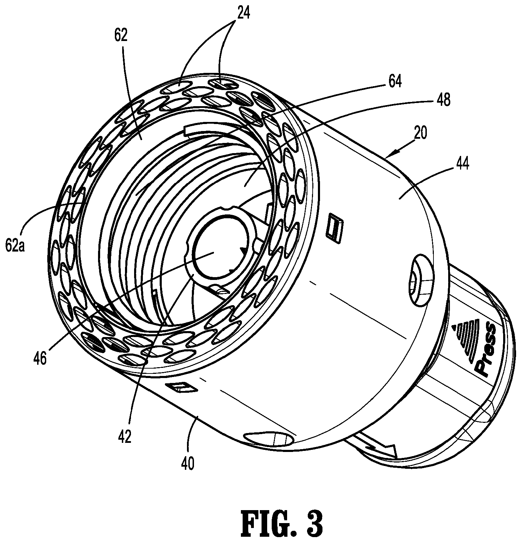

Referring to FIGS. 2-3, the shell assembly 20 includes a housing 40 having an inner housing portion 42 (FIG. 2A) and an outer housing portion 44. The inner housing portion 42 defines a central through bore 46. The central through bore 46 receives an anvil retainer (not shown) of the adaptor assembly 14 and the anvil center rod 30 when the stapling device 10 is approximated as is known in the art. The inner housing portion 42 and the outer housing portion 44 of the housing portion 44 define an annular channel 48.

The shell assembly 20 also includes a pusher assembly 50 and a knife assembly 52. The pusher assembly 50 and the knife assembly 52 are supported within the annular channel 48 of the shell housing 40 for movement between a retracted position (FIG. 2A) and an advanced position (FIG. 7). The pusher assembly 50 includes a pusher back 54 and a pusher 56. The pusher back 54 includes a proximal end that is coupled to the adaptor assembly 14 and a distal end that is in abutting relation to the pusher 56. The pusher 56 includes fingers 58 that are received within the staple receiving pockets 24 of the staple cartridge 22 and engage the staples 26. When the adaptor assembly 14 is driven to advance the pusher assembly 50 within the annular channel 48 of the shell housing 40, the fingers 58 of the pusher 56 are advanced through the staple receiving pockets 24 of the staple cartridge 22 to fire the staples 26 from the staple cartridge 22.

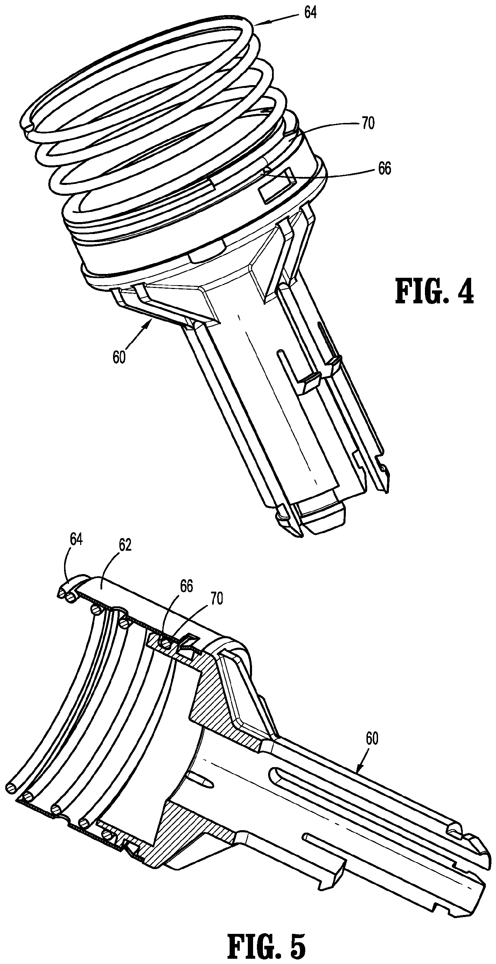

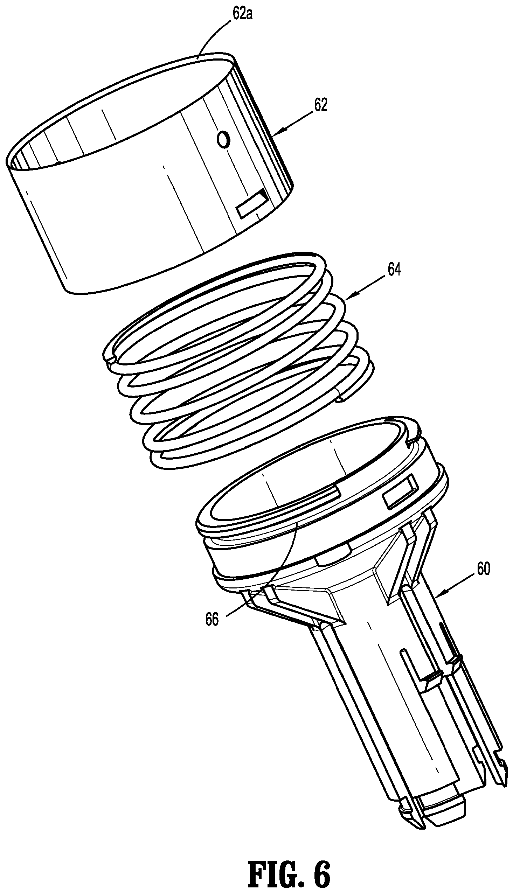

Referring also to FIGS. 4-6, the knife assembly 52 includes a knife carrier 60 and an annular knife blade 62. The knife carrier 60 includes a proximal end that is coupled to the adaptor assembly 14 and a distal end that supports the annular knife blade 62. In embodiments, the annular knife 62 includes a cutting edge 62a. The annular knife 62 is axially aligned with the cut ring 33 of the anvil head 28 of the anvil assembly 16. When the adaptor assembly 14 is driven to advance the knife carrier 60 within the annular channel 48 of the shell housing 40, the annular knife blade 62 is advanced into the cut ring 33 of the anvil assembly 16. For a more detailed description of the interconnection of the adaptor assembly 14 and the pusher and knife assemblies 50, 52 of a circular stapling device, see U.S. Patent Application Serial No. 15/467,153.

The knife assembly 52 also includes a biasing member 64 that is positioned on the distal portion of the knife carrier 60 and extends along an internal surface 62a (FIG. 2A) of the knife blade 62. In an unbiased state of the biasing member 64, a distal end of the biasing member 64 is positioned distally of the distal end of the annular knife blade 62 (FIG. 5) to shield the end of the knife blade 62 from a clinician. The biasing member 64 is in an unbiased state when the stapling device 10 is in an unapproximated state (FIG. 2A.)

In embodiments, the biasing member 64 is a coil spring and the knife carrier 60 includes an annular retainer groove 66 (FIG. 5). The annular retainer groove 66 is configured to receive a coil 70 at a proximal end of the biasing member 64 to secure the biasing member 64 to the knife carrier 60. As described above, the annular knife blade 62 is positioned about the biasing member 64 in a position to prevent separation of the coil 70 from the retaining groove 66.

Referring to FIG. 7, when the anvil assembly 16 and the shell assembly 20 are approximated, the distal end of the biasing member 64 is in contact with the cut ring 33. When the stapling device 10 is fired, the biasing member 64 and the annular knife 62 are advanced into the cut ring 33 to compress the biasing member 64 such that the distal end of the annular knife 62 moves distally beyond the distal end of the biasing member 64 and penetrates the cut ring 33 of the anvil assembly 16. When the knife assembly 52 is in the advanced position within the housing 40 of the shell assembly 20, the biasing member 64 is compressed between the cut ring 33 of the anvil assembly 16 and the knife carrier 60 to urge the knife assembly 52 to the retracted position and to urge the cut ring 33 to the advanced position. When the knife assembly 52 is retracted after firing of the stapling device 10, the biasing member 64 minimizes the likelihood that the cut ring 33 will stick to the annular knife 62 and be returned to its retracted position (FIG. 2A). As discussed above, if the cut ring 33 returns to its first position, the anvil head 28 may be prevented from moving to a tilted reduced profile position after firing.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

## Claims

1. A circular stapling device (10) comprising:
an elongate body (18) having a proximal portion and a distal portion;
a shell assembly (20) including a housing (40), a staple cartridge (22) supported on the housing, an annular knife (62) supported within the housing, and a biasing member (64) having a proximal portion and a distal portion, the annular knife being movable within the housing from a retracted position to an advanced position and extending from the distal portion of the housing in the advanced position, the biasing member extending to a position distally of the annular knife when the annular knife is in the retracted position; and
an anvil assembly (16) including a center rod (30) and an anvil head (28), the anvil head supporting a cut ring (33) that is axially aligned with the annular knife, the anvil assembly being movably supported in relation to the shell assembly between an unapproximated position and an approximated position;
**characterised in that** the biasing member is supported within the housing, wherein in the approximated position, the biasing member is engaged with the cut ring and urges the cut ring in a distal direction.

2. The circular stapling device (10) of claim 1, wherein the biasing member (64) is a coil spring.

3. The circular stapling device (10) of claim 2, wherein the shell assembly (20) includes a pusher assembly (50), the pusher assembly including a pusher (56) having a plurality of fingers (58), that is movable from a retracted position to an advanced position to eject staples (26) from the staple cartridge (22).

4. The circular stapling device (10) of any preceding claim, wherein the annular knife (62) includes a cutting edge that is positioned distally of the biasing member (64) when the annular knife is in the advanced position.

5. The circular stapling device (10) of any preceding claim, wherein the shell assembly (20) includes a knife carrier (60) that supports the annular knife (62), the knife carrier being movable from a retracted position to an advanced position within the housing (40), the annular knife engaging the cut ring (33) when the anvil assembly (16) and the shell assembly are in the approximated position and the annular knife is in the advanced position; preferably wherein the biasing member (64) is secured to the knife carrier; preferably wherein the knife carrier defines a retaining groove (66), the retaining groove receiving a coil (70) of the coil spring to secure the biasing member to the knife carrier.

6. The circular stapling device (10) of any preceding claim, further including a handle assembly (12), the elongate body (18) extending distally from the handle assembly.

7. The circular stapling device (10) of claim 3, wherein the shell assembly (20) includes a knife carrier (60) that supports the annular knife (62), the knife carrier being movable from a retracted position to an advanced position within the housing (40) independently of the pusher (56); preferably wherein the housing defines an annular channel (48), the pusher and the knife carrier being movably supported within the annular channel.

8. The circular stapling device (10) of claim 2, wherein the biasing member (64) is positioned within the annular knife (62).

9. A shell assembly (20) comprising:
a housing (40) having a proximal portion and a distal portion and defining an annular channel (48);
a staple cartridge (22) supported on the housing, the shell assembly defining a plurality of staple receiving pockets (24), each of the staple receiving pockets having a staple (26);
an annular knife (62) supported within the housing, the annular knife being movable within the housing from a retracted position to an advanced position, the annular knife extending from the distal portion of the housing when the annular knife is in the advanced position; and
a biasing member (64) supported within the annular channel of the housing, the biasing member extending to a position distally of the annular knife when the annular knife is in the retracted position;
**characterised in that** the biasing member is a coil spring.

10. The shell assembly (20) of claim 9, wherein the coil spring is positioned within the annular knife (62).

11. The shell assembly (20) of claim 9 or claim 10, wherein the shell assembly includes a pusher assembly (50), the pusher assembly including a pusher (56) having a plurality of fingers (58) that is movable from a retracted position to an advanced position to eject staples (26) from the staple cartridge (22).

12. The shell assembly (20) of claim 11, wherein the shell assembly includes a knife carrier (60) that supports the annular knife (62), the knife carrier being movable from a retracted position to an advanced position within the housing (40).

13. The shell assembly (20) of claim 12, wherein a cutting edge of the annular knife (62) is positioned distally of the biasing member (64) when the annular knife is in the advanced position.

14. The shell assembly (20) of claim 13, wherein the biasing member (64) is secured to the knife carrier (60).

15. The shell assembly (20) of claim 14, wherein the knife carrier (60) defines a retaining groove (66), the retaining groove receiving a coil (70) of the coil spring to secure the biasing member (64) to the knife carrier.

## Patentansprüche

1. Rundklammernahtgerät (10), Folgendes umfassend:
einen länglichen Körper (18) mit einem proximalen Abschnitt und einem distalen Abschnitt;
eine Schalenanordnung (20), die ein Gehäuse (40), eine an dem Gehäuse gelagerte Klammerpatrone (22), ein in dem Gehäuse gelagertes ringförmiges Messer (62) und ein Vorspannelement (64) mit einem proximalen Abschnitt und einem distalen Abschnitt aufweist, wobei das ringförmige Messer innerhalb des Gehäuses aus einer zurückgezogenen Position in eine vorgeschobene Position bewegbar ist und sich in der vorgeschobenen Position von dem distalen Abschnitt des Gehäuses erstreckt, wobei sich das Vorspannelement zu einer Position distal von dem ringförmigen Messer erstreckt, wenn sich das ringförmige Messer in der zurückgezogenen Position befindet; und
eine Ambossanordnung (16), die eine Mittelstange (30) und einen Ambosskopf (28) beinhaltet, wobei der Ambosskopf einen Schneidring (33) trägt, der axial mit dem ringförmigen Messer ausgerichtet ist, wobei die Ambossanordnung in Bezug auf die Schalenanordnung zwischen einer nicht angenäherten Position und einer angenäherten Position beweglich gehalten wird;
**dadurch gekennzeichnet, dass** das Vorspannelement innerhalb des Gehäuses gehalten wird, wobei das Vorspannelement in der angenäherten Position mit dem Schneidring in Eingriff steht und den Schneidring in eine distale Richtung drückt.

2. Rundklammernahtgerät (10) nach Anspruch 1, wobei das Vorspannelement (64) eine Schraubenfeder ist.

3. Rundklammernahtgerät (10) nach Anspruch 2, wobei die Schalenanordnung (20) eine Schubanordnung (50) beinhaltet, wobei die Schubanordnung einen Schieber (56) mit mehreren Fingern (58) beinhaltet, der von einer zurückgezogenen Position in eine vorgeschobene Position bewegt werden kann, um Klammern (26) aus der Klammerpatrone (22) auszuwerfen.

4. Rundklammernahtgerät (10) nach einem der vorhergehenden Ansprüche, wobei das ringförmige Messer (62) eine Schneidkante aufweist, die distal von dem Vorspannelement (64) angeordnet ist, wenn sich das ringförmige Messer in der vorgeschobenen Position befindet.

5. Rundklammernahtgerät (10) nach einem der vorhergehenden Ansprüche, wobei die Schalenanordnung (20) einen Messerträger (60) beinhaltet, der das ringförmige Messer (62) trägt, wobei der Messerträger aus einer zurückgezogenen Position in eine vorgeschobene Position innerhalb des Gehäuses (40) beweglich ist, wobei das ringförmige Messer in den Schneidring (33) eingreift, wenn die Ambossanordnung (16) und die Schalenanordnung in der angenäherten Position sind und das ringförmige Messer in der vorgeschobenen Position ist; wobei vorzugsweise das Vorspannelement (64) an dem Messerträger befestigt ist; wobei vorzugsweise der Messerträger eine Haltenut (66) definiert, wobei die Haltenut eine Windung (70) der Schraubenfeder aufnimmt, um das Vorspannelement an dem Messerträger zu befestigen.

6. Rundklammernahtgerät (10) nach einem der vorhergehenden Ansprüche, das ferner eine Griffanordnung (12) beinhaltet, wobei sich der längliche Körper (18) distal von der Griffanordnung erstreckt.

7. Rundklammernahtgerät (10) nach Anspruch 3, wobei die Schalenanordnung (20) einen Messerträger (60) beinhaltet, der das ringförmige Messer (62) trägt, wobei der Messerträger unabhängig von dem Schieber (56) aus einer zurückgezogenen Position in eine vorgeschobene Position innerhalb des Gehäuses (40) beweglich ist; vorzugsweise wobei das Gehäuse einen ringförmigen Kanal (48) definiert, wobei der Schieber und der Messerträger innerhalb des ringförmigen Kanals beweglich gelagert sind.

8. Rundklammernahtgerät (10) nach Anspruch 2, wobei das Vorspannelement (64) innerhalb des ringförmigen Messers (62) angeordnet ist.

9. Schalenanordnung (20), umfassend:
ein Gehäuse (40) mit einem proximalen Abschnitt und einem distalen Abschnitt, das einen ringförmigen Kanal (48) definiert;
eine Klammerpatrone (22), die von dem Gehäuse getragen wird, wobei die Schalenanordnung mehrere Klammeraufnahmetaschen (24) definiert, wobei jede der Klammeraufnahmetaschen eine Klammer (26) aufweist;
ein ringförmiges Messer (62), das innerhalb des Gehäuses getragen wird, wobei das ringförmige Messer innerhalb des Gehäuses von einer zurückgezogenen Position zu einer vorgeschobenen Position beweglich ist, wobei sich das ringförmige Messer von dem distalen Abschnitt des Gehäuses erstreckt, wenn das ringförmige Messer in der vorgeschobenen Position ist; und
ein Vorspannelement (64), das innerhalb des ringförmigen Kanals des Gehäuses getragen wird, wobei sich das Vorspannelement zu einer Position distal von dem ringförmigen Messer erstreckt, wenn sich das ringförmige Messer in der zurückgezogenen Position befindet;
**dadurch gekennzeichnet, dass** das Vorspannelement eine Schraubenfeder ist.

10. Schalenanordnung (20) nach Anspruch 9, wobei die Schraubenfeder innerhalb des ringförmigen Messers (62) angeordnet ist.

11. Schalenanordnung (20) nach Anspruch 9 oder Anspruch 10, wobei die Schalenanordnung eine Schubanordnung (50) beinhaltet, wobei die Schubanordnung einen Schieber (56) mit mehreren Fingern (58) beinhaltet, der von einer zurückgezogenen Position in eine vorgeschobene Position beweglich ist, um Klammern (26) aus der Klammerpatrone (22) auszuwerfen.

12. Schalenanordnung (20) nach Anspruch 11, wobei die Schalenanordnung einen Messerträger (60) beinhaltet, der das ringförmige Messer (62) trägt, wobei der Messerträger von einer zurückgezogenen Position in eine vorgeschobene Position innerhalb des Gehäuses (40) beweglich ist.

13. Schalenanordnung (20) nach Anspruch 12, wobei eine Schneidkante des ringförmigen Messers (62) distal des Vorspannelements (64) angeordnet ist, wenn sich das ringförmige Messer in der vorgeschobenen Position befindet.

14. Schalenanordnung (20) nach Anspruch 13, wobei das Vorspannelement (64) an dem Messerträger (60) befestigt ist.

15. Schalenanordnung (20) nach Anspruch 14, wobei der Messerträger (60) eine Haltenut (66) definiert, wobei die Haltenut eine Spule (70) der Schraubenfeder aufnimmt, um das Vorspannelement (64) an dem Messerträger zu befestigen.

## Revendications

1. Dispositif d'agrafage circulaire (10) comprenant :
un corps allongé (18) ayant une partie proximale et une partie distale ;
un ensemble coque (20) comportant un boîtier (40), une cartouche d'agrafes (22) supportée sur le boîtier, un couteau annulaire (62) supporté à l'intérieur du boîtier, et un élément de sollicitation (64) ayant une partie proximale et une partie distale, le couteau annulaire étant mobile à l'intérieur du boîtier d'une position rétractée à une position avancée et s'étendant depuis la partie distale du boîtier dans la position avancée, l'élément de sollicitation s'étendant vers une position de manière distale par rapport au couteau annulaire lorsque le couteau annulaire est dans la position rétractée ; et
un ensemble enclume (16) comportant une tige centrale (30) et une tête d'enclume (28), la tête d'enclume supportant une bague de coupe (33) qui est alignée axialement avec le couteau annulaire, l'ensemble enclume étant supporté de manière mobile par rapport à l'ensemble coque entre une position non rapprochée et une position rapprochée ;
**caractérisé en ce que** l'élément de sollicitation est supporté à l'intérieur du boîtier, dans la position rapprochée, l'élément de sollicitation étant mis en prise avec la bague de coupe et poussant la bague de coupe dans une direction distale.

2. Dispositif d'agrafage circulaire (10) selon la revendication 1, dans lequel l'élément de sollicitation (64) est un ressort hélicoïdal.

3. Dispositif d'agrafage circulaire (10) selon la revendication 2, dans lequel l'ensemble coque (20) comporte un ensemble poussoir (50), l'ensemble poussoir comportant un poussoir (56) ayant une pluralité de doigts (58), qui est mobile d'une position rétractée à une position avancée pour éjecter des agrafes (26) de la cartouche d'agrafes (22).

4. Dispositif d'agrafage circulaire (10) selon l'une quelconque des revendications précédentes, dans lequel le couteau annulaire (62) comporte un bord coupant qui est positionné de manière distale par rapport à l'élément de sollicitation (64) lorsque le couteau annulaire est dans la position avancée.

5. Dispositif d'agrafage circulaire (10) selon l'une quelconque des revendications précédentes, dans lequel l'ensemble coque (20) comporte un support de couteau (60) qui supporte le couteau annulaire (62), le support de couteau étant mobile d'une position rétractée à une position avancée à l'intérieur du boîtier (40), le couteau annulaire venant en prise avec la bague de coupe (33) lorsque l'ensemble enclume (16) et l'ensemble coque sont dans la position rapprochée et que le couteau annulaire est dans la position avancée ; de préférence dans lequel l'élément de sollicitation (64) est fixé au support de couteau ; de préférence dans lequel le support de couteau définit une rainure de retenue (66), la rainure de retenue recevant une bobine (70) du ressort hélicoïdal pour fixer l'élément de sollicitation au support de couteau.

6. Dispositif d'agrafage circulaire (10) selon l'une quelconque des revendications précédentes, comportant en outre un ensemble poignée (12), le corps allongé (18) s'étendant de manière distale depuis l'ensemble poignée.

7. Dispositif d'agrafage circulaire (10) selon la revendication 3, dans lequel l'ensemble coque (20) comporte un support de couteau (60) qui supporte le couteau annulaire (62), le support de couteau étant mobile d'une position rétractée à une position avancée à l'intérieur du boîtier (40) indépendamment du poussoir (56) ; de préférence dans lequel le boîtier définit un canal annulaire (48), le poussoir et le support de couteau étant supportés de manière mobile à l'intérieur du canal annulaire.

8. Dispositif d'agrafage circulaire (10) selon la revendication 2, dans lequel l'élément de sollicitation (64) est positionné à l'intérieur du couteau annulaire (62).

9. Ensemble coque (20) comprenant :
un boîtier (40) ayant une partie proximale et une partie distale et définissant un canal annulaire (48) ;
une cartouche d'agrafes (22) supportée sur le boîtier, l'ensemble coque définissant une pluralité de poches de réception d'agrafes (24), chacune des poches de réception d'agrafes ayant une agrafe (26) ;
un couteau annulaire (62) supporté à l'intérieur du boîtier, le couteau annulaire étant mobile à l'intérieur du boîtier d'une position rétractée à une position avancée, le couteau annulaire s'étendant depuis la partie distale du boîtier lorsque le couteau annulaire est dans la position avancée ; et
un élément de sollicitation (64) supporté à l'intérieur du canal annulaire du boîtier, l'élément de sollicitation s'étendant vers une position de manière distale par rapport au couteau annulaire lorsque le couteau annulaire est dans la position rétractée ;
**caractérisé en ce que** l'élément de sollicitation est un ressort hélicoïdal.

10. Ensemble coque (20) selon la revendication 9, dans lequel le ressort hélicoïdal est positionné à l'intérieur du couteau annulaire (62).

11. Ensemble coque (20) selon la revendication 9 ou la revendication 10, dans lequel l'ensemble coque comporte un ensemble poussoir (50), l'ensemble poussoir comportant un poussoir (56) ayant une pluralité de doigts (58) qui est mobile d'une position rétractée à une position avancée pour éjecter des agrafes (26) de la cartouche d'agrafes (22).

12. Ensemble coque (20) selon la revendication 11, dans lequel l'ensemble coque comporte un support de couteau (60) qui supporte le couteau annulaire (62), le support de couteau étant mobile d'une position rétractée à une position avancée à l'intérieur du boîtier (40).

13. Ensemble coque (20) selon la revendication 12, dans lequel un bord coupant du couteau annulaire (62) est positionné de manière distale par rapport à l'élément de sollicitation (64) lorsque le couteau annulaire est dans la position avancée.

14. Ensemble coque (20) selon la revendication 13, dans lequel l'élément de sollicitation (64) est fixé au support de couteau (60).

15. Ensemble coque (20) selon la revendication 14, dans lequel le support de couteau (60) définit une rainure de retenue (66), la rainure de retenue recevant une bobine (70) du ressort hélicoïdal pour fixer l'élément de sollicitation (64) au support de couteau.
